# DEMANDE DE BREVET EUROPEEN

(11) **EP 2 131 188 A1**
(43) Date de publication de la demande: **09.12.2009**
(21) Numéro de dépôt: 09290414.3
(22) Date de dépôt: 05.06.2009
(51) Int. Cl.: G01N 27/407

(54) **Capteur de gaz pour monoxyde de carbone dans une atmosphère réductrice**

(30) Priorité: 06.06.2008 FR 0803150
(71) Demandeur: ASSOCIATION POUR LA RECHERCHE ET LE DEVELOPPEMENT DES METHODES ET PROCESSUS INDUSTRIELS (ARMINES), F-75272 Paris Cédex 06 (FR)
(72) Inventeur: Pijolat, Christophe, 42290 Sorbiers (FR); Viricelle, Jean-Paul, 41260 Andrezieux-Boutheon (FR); Tournier, Guy, 42023 Saint-Etienne (FR)
(74) Mandataire: Lavialle, Bruno François Stéphane

(57) **Abrégé**

Capteur potentiométrique comportant une première électrode (1) et une deuxième électrode (2) entre lesquelles s'étend un électrolyte (3), la première électrode étant en un métal réactif au monoxyde de carbone, la deuxième électrode étant en un métal ayant une réactivité plus faible à l'oxyde de carbone que la première électrode, **caractérisé en ce que** l'électrolyte est en cérine dopée au samarium ou au gadolinium.

## Description

La présente invention concerne un capteur de monoxyde de carbone, plus particulièrement adapté à la détection des faibles concentrations de monoxyde de carbone dans une atmosphère réductrice comme une atmosphère pauvre en oxygène, présentant plus particulièrement des traces d'oxygène inférieures à environ 100 ppm, telle qu'une atmosphère riche en dihydrogène (H₂) comme celle rencontrée dans les piles à combustible.

### ARRIERE PLAN DE L'INVENTION

L'invention est plus particulièrement destinée à être utilisée avec des piles à combustible comportant une membrane d'échange de protons (pile à combustible dite "PEM" de l'anglais "Proton Exchange Membrane"). De telles piles à combustible utilisent le platine comme catalyseur, en particulier sur l'anode, pour dissocier les molécules de dihydrogène. Or, il est connu que le monoxyde de carbone interagit avec le platine et diminue les performances de ces piles à combustible. Il est donc nécessaire de détecter la présence de monoxyde de carbone dans le dihydrogène. Il a été envisagé d'utiliser comme capteur de monoxyde de carbone dans le dihydrogène des reproductions à échelle réduite de piles à combustible de type PEM ou SOFC ("Solid Oxide Fuel Cell" ou pile à combustible à oxyde solide) et de détecter la baisse de performance due à la présence de monoxyde de carbone dans ces piles à combustible. Cette solution est cependant de mise en oeuvre délicate. Il a également été envisagé d'utiliser des capteurs à base de chlorure de cuivre ou de bromure de cuivre mais de tels capteurs ont une sensibilité à l'humidité trop importante pour une application à des piles à combustible.

### OBJET DE L'INVENTION

Un but de l'invention est donc de proposer un moyen pour la détection du monoxyde de carbone dans une atmosphère riche en dihydrogène.

### RESUME DE L'INVENTION

A cet effet, on prévoit, selon l'invention, un capteur potentiométrique comportant une enceinte qui est pourvue de moyens de passage d'un gaz et qui reçoit une première électrode et une deuxième électrode entre lesquelles s'étend un électrolyte, la première électrode étant en un métal réactif au monoxyde de carbone et la deuxième électrode étant en un métal ayant une réactivité plus faible à l'oxyde de carbone que la première électrode, l'électrolyte étant en cérine dopée au samarium ou au gadolinium.

On s'est aperçu qu'un capteur avec un tel électrolyte et les deux électrodes soumises au gaz était particulièrement sensible aux faibles concentrations de monoxyde de carbone en l'absence d'oxygène et présentait des performances stables dans les atmosphères riches en dihydrogène.

De préférence, la première électrode est en platine, et avantageusement, la deuxième électrode est en or.

Cette structure est particulièrement efficace.

L'invention a également pour objet l'utilisation d'un tel capteur pour la détection d'oxyde de carbone dans une atmosphère pauvre en oxygène et plus particulièrement présentant des traces d'oxygène inférieures à environ 100 ppm.

Ce capteur est particulièrement efficace dans ces conditions.

### BREVE DESCRIPTION DES DESSINS

D'autres caractéristiques et avantages de l'invention ressortiront à la lecture de la description qui suit d'un mode de réalisation particulier non limitatif de l'invention.

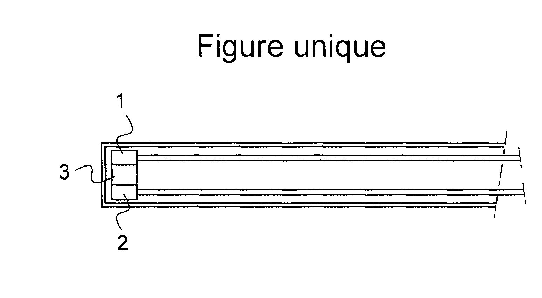

Il sera fait référence à la figure unique annexée représentant schématiquement et en coupe longitudinale un capteur conforme à l'invention.

### DESCRIPTION DETAILLEE DE L'INVENTION

Le capteur comprend une enceinte 1 pourvue de moyens non représentés de passage d'un gaz et recevant une première électrode 1 et une deuxième électrode 2 entre lesquelles s'étend un électrolyte 3. Comme cela est visible sur la figure, l'enceinte est agencée de telle manière que les deux électrodes soient soumises à la même atmosphère, c'est-à-dire l'atmosphère à analyser.

La première électrode 1 est formée de platine, la deuxième électrode 2 est formée d'or, les électrodes 1 et 2 étant reliées à un dispositif de mesure de tension non représenté.

L'électrolyte 5 est formé d'un substrat en alumine sur lequel est déposée une couche de cérine (oxyde de cérium) dopée au samarium. La cérine est ici dopée à 15 % environ avec du samarium, la teneur en samarium pouvant être comprise entre un ou quelques pour cent jusqu'à quelques dizaines de pour cent.

Les électrodes 1 et 2 sont formées par dépôt de métal en deux points opposés de l'électrolyte 3.

L'utilisation de ce capteur est classique et ne sera donc pas détaillée ici.

Ce capteur présente des performances particulièrement satisfaisantes pour des températures comprises entre 200°C et 300°C environ.

Pour maintenir le capteur dans cette plage de températures, il est possible de prévoir sur l'électrolyte une résistance chauffante, par exemple, en platine.

Bien entendu, l'invention n'est pas limitée au mode de réalisation décrit et on peut y apporter des variantes de réalisation sans sortir du cadre de l'invention tel que défini par les revendications.

En particulier, bien que la première électrode et la deuxième électrode aient été décrites constituées de platine et d'or respectivement, il est possible d'utiliser tout couple de matériaux conducteurs présentant des réactivités différentes au monoxyde de carbone.

D'autres matériaux que l'alumine sont utilisables pour former le substrat de l'électrolyte 3. L'électrolyte 3 peut également être formé majoritairement de cérine dopée au samarium (le matériau du substrat étant minoritaire à l'électrolyte comportant un barreau monolithique de cérine dopée au samarium).

Dans une variante un peu moins performante, l'électrolyte peut également être formé de cérine dopée au gadolinium, par exemple à 10 % de gadolinium environ, la teneur en gadolinium pouvant être comprise entre quelques pour cent et quelques dizaines de pour cent.

## Revendications

1. Capteur potentiométrique comportant une enceinte qui est pourvue de moyens de passage d'un gaz et qui reçoit une première électrode (1) et une deuxième électrode (2) entre lesquelles s'étend un électrolyte (3), la première électrode étant en un métal réactif au monoxyde de carbone, la deuxième électrode étant en un métal ayant une réactivité plus faible à l'oxyde de carbone que la première électrode et l'électrolyte étant en cérine dopée au samarium ou au gadolinium.

2. Capteur selon la revendication 1, dans laquelle la première électrode (1) est en platine.

3. Capteur selon la revendication 1 ou la revendication 2, dans lequel la deuxième électrode (2) est en or.

4. Capteur selon l'une quelconque des revendications 1 à 3, dans lequel les métaux de la première électrode (1) et de la deuxième électrode (2) sont déposés en deux points opposés de l'électrolyte (3).

5. Capteur selon l'une quelconque des revendications 1 à 4, dans lequel la cérine se présente sous la forme d'une couche déposée sur un substrat.

6. Capteur selon la revendication 5, dans lequel le substrat est en alumine.

7. Capteur selon la revendication 1, dans lequel le samarium ou le gadolinium est présent dans la cérine à une teneur de l'ordre de quelques pour cent à quelques dizaines de pour cent.

8. Capteur selon la revendication 7, dans lequel la teneur de samarium est de 15 pour cent environ.

9. Capteur selon la revendication 7, dans lequel la teneur de gadolinium est de 10 pour cent environ.

10. Utilisation d'un capteur conforme à l'une quelconque des revendications précédentes pour la détection d'oxyde de carbone dans une atmosphère pauvre en oxygène et plus particulièrement présentant des traces d'oxygènes inférieures à environ 100 ppm.
